(19) Europäisches Patentamt
European Patent Office
Office européen des brevets

(11) Numéro de publication: **0 331 564**
**A2**

(12) # DEMANDE DE BREVET EUROPEEN

(21) Numéro de dépôt: **89400525.5**

(22) Date de dépôt: **24.02.89**

(51) Int. Cl.4: **C 08 B 37/00**
C 12 P 19/26, A 23 C 9/137,
A 61 K 31/715

(30) Priorité: **26.02.88 FR 8802405**

(43) Date de publication de la demande:
**06.09.89 Bulletin 89/36**

(84) Etats contractants désignés:
**AT BE CH DE ES FR GB GR IT LI LU NL SE**

(71) Demandeur: **SOCIETE DE DEVELOPPEMENTS ET D'INNOVATIONS DES MARCHES AGRICOLES ET ALIMENTAIRES - SODIMA- UNION DES COOPERATIVES AGRICOLES**
**170 bis, Boulevard du Montparnasse**
**F-75014 Paris (FR)**

(72) Inventeur: **Doco, Thierry**
**16, rue Paul Bourget**
**F-62000 Dainville (FR)**

**Fournet, Bernard**
**21, rue du Moulin d'Ascq**
**F-59650 Villeneuve D'Ascq (FR)**

**Carcano, Didier**
**44, rue Poliveau**
**F-75005 Paris (FR)**

**Ramos, Patricia**
**25, rue du 18 juin 1940**
**F-94700 Maisons-Alfort (FR)**

**Loones, Alain**
**33, rue des Perroquets**
**F-94350 Villiers Sur Marne (FR)**

**Piot, Jean Marie**
**5 Avenue du Vieux Chateau**
**F-59650 Villeneuve d'Asco (FR)**

**Guillochon, Didier**
**126, rue Poincaré**
**F-59000 Marcq en Baroeuil (FR)**

(74) Mandataire: **Warcoin, Jacques et al**
**Cabinet Régimbeau 26, avenue Kléber**
**F-75116 Paris (FR)**

Le (Les) microorganisme(s) a (ont) été déposé(s) auprès CNCM sous le(s) numéro(s) I-733, I-734, I-735.

(54) **Polysaccharide, application comme agent épaississant et comme agent antitumoral.**

(57) La présente invention concerne un nouveau polysaccharide de masse moléculaire de l'ordre du million constitué par la répétition d'unité tétrasaccharidique composant de galactose, glucose et N-acétylgalactosamine dans les rapports molaires 2:1:1 respectivement. Ce polysaccharide est utilise à titre d'agent de texture épaississant en particulier pour des produits du type lait fermenté tel que le yaourt. Il est également utile comme médicament à titre d'agent antitumoral.

Ce polysaccharide peut être produit par la bactérie Streptococcus thermophilus mise en culture dans les mêmes conditions que les conditions de fabrication du yaourt.

EP 0 331 564 A2

## Description

### POLYSACCHARIDE, APPLICATION COMME AGENT EPAISSISSANT ET COMME AGENT ANTITUMORAL.

La présente invention concerne un nouveau polysaccharide. La présente invention concerne également l'application de ce polysaccharide comme agent de texture épaississant d'un produit de type lait fermenté, notamment d'un yaourt et comme médicament à titre de composé antitumoral. La présente invention concerne encore un procédé de production dudit polysaccharide.

Le produit que l'on désigne sous le nom de yaourt correspond à un aliment du type lait fermenté préparé par acidification du lait à l'aide de deux ferments lactiques, les bactéries Streptococcus thermophilus et Lactobacillus bulgaricus. Peuvent également rentrer dans la composition du yaourt des consitutants tels que de la poudre de lait, du sucre ou des adjuvants fruités. En revanche, l'adjonction d'autres composés exogènes est déconseillée pour que soient conservées les qualités de naturel et organoleptiques du produit. En particulier, l'adjonction d'agents de texture épaississants exogènes, tels que certains polysaccharides extraits des algues ou dérivés de la cellulose comme la CMC (carboxy-méthyl-cellulose) employés dans l'alimentation, notamment les desserts, les sauces, etc., est à éviter.

On distingue trois catégories de yaourts selon leur texture :

. les yaourts liquides ou à boire,
. les yaourts brassés, produit lié, onctueux, souple et lisse mais d'une certaine épaisseur,
. les yaourts fermes, d'aspect plus gélatineux.

Le yaourt liquide est le produit naturellement obtenu. Pour préparer un yaourt brassé ou ferme, il est nécessaire d'ajouter dans la préparation de la poudre de lait écrémé qui constitue un apport de matière sèche et de protéines donnant sa texture épaisse au yaourt. Le yaourt ferme se prépare directement par fermentation en petit pot avec appoint en poudre de lait. Le yaourt brassé, en revanche, est préparé par fermentation en cuve, par exemple des cuves de 3000 l. Après culture, le produit devient épais, il est soutiré de la cuve, pompé, refroidi et conditionné en petit pot. Il perd, au cours de ces opérations mécaniques, une grande partie de sa texture épaisse.

Les études réalisées auprès des consommateurs révèlent leur goût prononcé pour ces yaourts du type brassé, souple et lisse avec toutefois le souhait que ce type de produit puisse présenter une consistance plus épaisse.

La présente invention vise donc entre autres à satisfaire à cette demande en proposant un yaourt à texture plus épaisse qui conserve les qualités de naturel et organoleptique du produit.

Pour ce faire, il n'est pas possible d'augmenter la quantité de poudre de lait rentrant dans la préparation du yaourt, au-delà de 5 % en fonction de limitations légales d'une part, mais, d'autre part, justifié par des considérations d'ordre organoleptique.

Dans US 4 396 763, il a été décrit un polysaccharide produit à partir de bactéries lactiques cultivées dans du lactosérum ou du petit lait et non du lait, présentant une activité antitumorale. Ce polysaccharide était composé exclusivement de glucose et galactose. Une activité antitumorale a été mise en évidence, cependant aucune propriété épaississante n'a été démontrée.

Dans Biotechnology letters (Vol. 8, n° 9, 1986, pages 625-628 CERNING et Coll.), un polysaccharide composé de galactose, glucose et rhamnose dans un rapport 4:1:1 de masse moléculaire 500.000, produit par la bactérie Lactobacillus bulgaricus a été décrit. Aucune propriété épaississante n'a été démontrée.

Aucun des polysaccharides de bactéries lactiques décrits jusqu'à ce jour n'ont été complétement caractérisés quant à leur structure chimique. En outre il n'a pas été proposé d'ajouter ces polysaccharides à un yaourt pour l'épaissir.

A fortiori, il n'a pas été décrit d'ajouter à un yaourt un polysaccharide produit par Streptococcus thermophilus dans un milieu et des conditions de cultures identiques à ceux qui en présence des deux bactéries du yaourt des streptococcus thermophilus et Lactobacillus bulgaricus, conduiraient à un yaourt.

Le but de la présente invention était d'obtenir un poly saccharide produit par une bactérie lactique du type Streptococcus thermophilus qui puisse être caractérisé et servir comme agent de texture épaississant notamment de yaourt en préservant au maximum toutes les qualités naturelles et organoleptiques du produit et enfin qui présente une activité antitumorale.

Pour ce faire, la présente invention a donc pour objet un polysaccharide de masse moléculaire comprise entre 800 000 et 1 million de daltons constitué par la répétition d'une unité tétrasaccharidique composée de D-galactose, D-glucose et D-N-acétylgalactosamine dans les rapports molaires 2:1:1 respectivement.

Plus précisément, ce polysaccharide répond à la formule générale suivante :

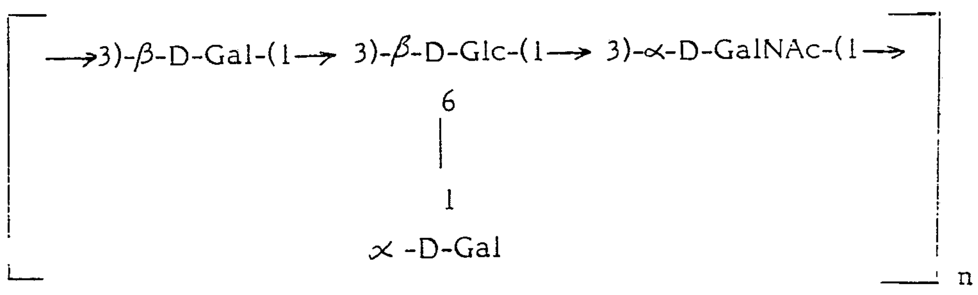

Le polysaccharide peut encore être présenté par la formule développée suivante :

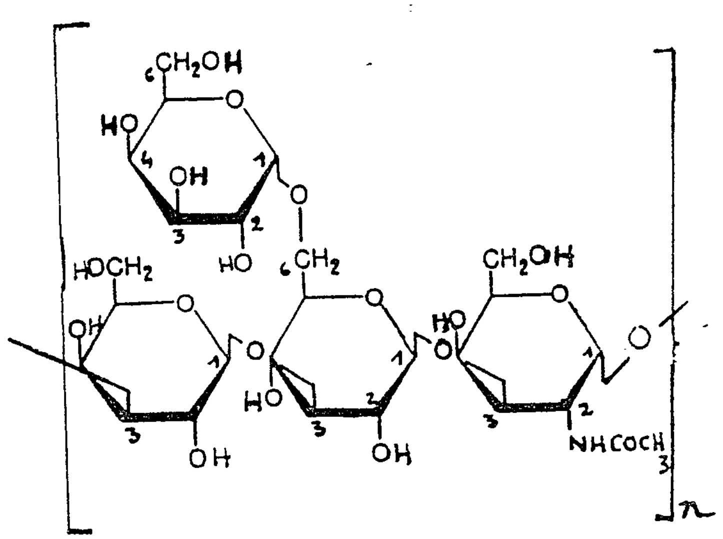

Ce polysaccharide est obtenu après extraction et purification de l'exopolysaccharide sécrété sous forme de gel dans un milieu de culture de la bactérie Streptococcus thermophilus. Le milieu et les conditions de culture sont identiques à ceux qui conduisent en présence de Streptococcus thermophilus et Lactobacillus bulgaricus à un yaourt. Il s'agit d'un milieu comportant du lait écrémé, par exemple du lait écrémé reconstitué stérilisé avec un inoculum de 3 % en poids de ferment Streptococcus thermophilus, la fermentation était réalisée à 43°C jusqu'à pH 5, soit environ 4 heures.

Les souches bactériennes utilisées ont été sélectionnées en fonction de leur caractère épaississant, c'est-à-dire de leur faculté de produire ce polysaccharide, en l'occurence le polysaccharide a été produit à partir des souches bactériennes référencées CNCM I-733, CNCM I-734, CNCM I-735.

Plus généralement, le milieu et les conditions de culture peuvent être du lait fermenté avec 1 à 5 % en poids de ferment lactique à une température de 30 à 45°C, de préférence 40 à 45°C, pendant une durée de 3 à 5 Heures. Le lait peut être du lait naturel, mais également du lait reconstitué. Il peut s'agir de lait écrémé ou encore de lait stérilisé.

Lorsque la culture est terminée, le milieu est divisé en une fraction liquide et une fraction bactérienne. A partir de ces deux fractions, le polysaccharide peut être obtenu par des opérations d'isolement et d'extraction connues de l'homme de l'art, telles que filtration sur gel, chromatographie par échange d'ions, relargage, dissolution fractionnée, dialyse. Ces techniques peuvent être utilisées séparément ou prises en combinaison.

Toutefois, la présente invention a également pour objet un nouveau procédé d'isolement de l'exopolysaccharide produit par Streptococcus thermophilus conformément à la présente invention, caractérisé en ce qu'on réalise une hydrolyse protéolitique en continu dans un réacteur enzymatique, l'action de l'enzyme étant maintenu par l'apport de lait fermenté en solution dans le tampon d'hydrolyse et on élimine les produits d'hydrolyse et concentre le polysaccharide à l'aide d'une unité d'ultrafiltration.

Ce polysaccharide est un gel visqueux particulièrement épaississant qui, compte tenu de ses conditions de culture, de ses qualités rhéologiques, en fonction du souci de préserver au maximum les qualités naturelles et organoleptiques d'un yaourt, s'avère particulièrement intéressant comme agent épaississant pour yaourt. En effet, ce polysaccharide offre toutes les garanties d'un produit endogène dans la mesure où il est produit à

partir d'une des bactéries du yaourt dans un milieu et des conditions de culture identiques à celles conduisant au yaourt en présence des deux bactéries lactiques du yaourt.

En outre, comme il sera vu plus loin, ce polysaccharide présente des propriétés antitumorales notables.

La présente invention a donc aussi pour objet un procédé d'épaississement de produit de type lait fermenté, notamment de yaourt, caractérisé en ce qu'on ajoute au dit produit une quantité, notamment, mais de manière limitative, de 0,1 à 0,5 %, du polysaccharide selon l'invention.

Ce polysaccharide peut aussi être employé d'une manière générale comme agent de texture épaississant par exemple dans d'autres produits lactés et diététiques que des yaourts, notamment des desserts, sauces et autres produits alimentaires.

Enfin, ce polysaccharide compte tenu de ses propriétés antitumorales, peut être utilisé à titre de médicament.

D'autres caractéristiques et avantages de la présente invention apparaîtront à la lumière des exemples qui vont suivre.

Sur la figure 1 est représentée une courbe de détermination de la masse moléculaire du polysaccharide Streptococcus thermophilus par chromatographie de gel filtration sur Sépharose 4-B.

Sur la figure 2 est représenté un spectre de résonance magnétique nucléaire de C-13 du polysaccharide (50 mg) de Streptococcus thermophilus CNCM I-733.

Sur la figure 3, est représenté un spectre de chromatographie HPLC d'un lait reconstitué stérilisé ensemencé par Streptococcus thermophilus CNCM I-735 soniqué et filtré, injecté sur colonne couplée d'AX-300 et TSK-250. Détection UV 206 nm (A) et réfractométrique (B). Le diagramme (C) correspond à la chromatographie HPLC du polysaccharide isolé par hydrolyse pronasique.

Sur la figure 4 sont représentés les groupes de viscosité d'un yaourt avec (0,025 %) et sans polysaccharide.

La figure 5 représente le schéma d'un réacteur enzymatique couplé à la cellule d'ultrafiltration utile pour le procédé d'isolement du polysaccharide.

La figure 6 représente la courbe d'hydrolyse d'un réacteur de la figure 5.

La figure 7 représente le profil d'élution obtenu par chromatographie sur DEAE Tris-Acryl pour la purification du polysaccharide obtenu par le procédé d'isolement dans un réacteur enzymatique.

La figure 8 représente la chromatographie du pic polysaccharidique sur Sépharose 4B pour la purification du polysaccharide obtenu par isolement dans un réacteur enzymatique.

EXEMPLE 1 - Polysaccharide produit par Streptococcus thermophilus cultivé sur milieu lacté.

1. Souche utilisée

Les souches utilisées sont de l'espèce Streptococcus thermophilus, elles proviennent de ferments commerciaux SODIMA pour yaourt brassé. Ces souches sont conservées à l'état congelé dans l'azote liquide (-196°C). Elles sont référencées dans la collection particulière du Centre National de Culture de Microorganisme de l'Institut Pasteur, 25 rue du Docteur Roux - 75724 PARIS CEDEX 15, où elles ont été déposées le 12 février 1988.

Ont été utilisées trois souches différentes provenant des ferments SODIMA 53 (CNCM I-733), SODIMA 822 (CNCM I-735), SODIMA 413 (CNCM I-734).

2. Conditions de culture

Après une préculture sur 10 ml de lait écrémé reconstitué stérilisé, une inoculation de 1 % en poids de ferments lactiques ($10^7$ à $10^9$ germe/ml d'inoculum) est réalisée sur un lait reconstitué à 147 g/l écrémé et stérilisé. La fermentation est réalisée à 43°C jusqu'à pH 5, puis le produit est conditionné à chaud après lissage au laboratoire dans des pots stérilisés de 125 g. Ces pots sont immédiatement refroidis en chambre froide, puis stockés à 7°C. Le lendemain, les échantillons sont expédiés en condition, réfrigérés au laboratoire pour extraction du polysaccharide.

3. Extraction du polysaccharide

1 litre de lait fermenté par Streptococcus thermophilus est dialysé 3 jours à + 4°C contre de l'eau désionisée. L'adialysable est ensuite lyophilisé. Le lyophilisat, repris par un tampon acétate de calcium 10 mM à pH 8 à la concentration de 10 p.cent, est hydrolysé par la pronase (Calbiochem, Enzyme/substrat : 1/50, 37°C, 48h) en présence de toluène pour inhiber la croissance bactérienne. L'hydrolysat est amené à pH 4,5 par de l'acide acétique et le matériel macromoléculaire précipité par 10 volumes d'éthanol. Ce protocole expérimental est répété deux fois. A la fin de la troisième hydrolyse, la protéine enzymatique est précipitée par l'acide trichloracétique à 10 % (vol/vol). Après centrifugation, le surnageant est purifié par passage sur résine échangeuse de cations (Dowex 50x8) et d'anions (Dowex 1x8). Après concentration, le matériel macromoléculaire est précipité par 10 volumes d'éthanol. Après centrifugation, le précipité est repris par de l'eau et lyophilysé.

Le polysaccharide est purifié par chromatographie de gel filtration (20 à 25 mg de précipité pronasique dans 1 ml d'acétate d'ammonium 100 mM) sur colonne de Séphacryl S-1000 (50x1,6 cm, Pharmacia). L'élution est réalisée pa de l'acétate d'ammonium 100 mM à débit de 1 ml/min. Le matériel glucidique est détecté par réfractométrie.

4. Masse moléculaire

La masse moléculaire du polysaccharide est estimée par chromatographie de gel filtration effectuée sur une colonne (92 x 1,8 cm) de Sépharose-4B étalonnée à l'aide de dextrans $5.10^6$ ; $5.10^5$ ; $2,3.10^5$ ; $4.10^4$ ; $1.10^4$ (Sigma) et de lactose. La chromatographie est développée à l'aide d'un tampon phosphate 5mM, pH 6,15 à 15 ml/h. La détection est effectuée à l'aide d'un réfractomètre.

5. Composition en monosaccharides du polysaccharide

Les rapports molaires en monosaccharides sont effectués

- après hydrolyse trifluoroacétique (ATFA 4N,4h,100° C) par chromatographie en phase gazeuse des polyols acétates sur colonne capilliare (0,32 mm x 30 m) de silicone OV 101, température programmée de 130° C à 240° C à raison de 2° C/min. et par chromatographie d'exclusion d'ions sur colonne d'HPX-87H (Biorad, 30x0,78 cm) éluée par de l'acide sulfurique 0,008N à un débit de 0,6 ml/min. Les monosaccharides sont détectés par réfractométrie.
- après méthanolyse (MeOH/HCl 0,5N,80° C,24h) par chromatographie en phase gazeuse sur colonne (0,3x300 cm) de silicone OV 210 des méthylglycosides trifluoroacétylés selon le protocole expérimental de Zanetta et al. (1).

6. Méthylation

Le polysaccharide (0,5 mg) est méthylé par la méthode de Paz-Parente et al. (2) au lithium méthyl sulfinyl carbanion, iodure de méthyle et les éthers méthyliques identifiés par chromatographie en phase gazeuse couplée au spectromètre de masse selon la méthode de Fournet et al. (3).

7. Résonance magnétique nucléaire du C-13

Le spectre de résonance magnétique nucléaire du C-13 à 100.614 MHz en abondance naturelle a été obtenu sur un sprectromètre BRUKER AM-400 WB couplé à un calculateur ASPECT 3000 (Centre Commun de Mesures, USTL-Flandres-Artois). L'échantillon (50 mg/1 ml $D_2O$) a été analysé à 80° C en utilisant le programme standard POWGATE.AU (1H Broad-band with WALTZ decoupling). $D_1$ = délai de préparation = 0.1s ; PW = 90° = 6µs ; $S_1 = S_2 = 1$ Watt.

La fenêtre spectrale est de 26000 Hz pour 32K (SI = TD) ce qui nous donne un temps d'acquisition de 0.623s et une résolution de 1.606Hz par point. Le nombre total d'acquisition est de 16800. Les déplacements chimiques sont mesurés par rapport au TMSP-$D_4$ (Triméthylsilyl-2,2',3,3'-D-Propionate de sodium) utilisé comme référence interne ($\delta_{CH3}$ = O.O ppm), avec une précision de l'ordre de 0.1 ppm.

8. Caractérisation du polysaccharide

Le traitement d'un litre de culture lactée conduit à l'obtention de 40 mg de polysaccharide en moyenne. La chromatographie de gel filtration sur Sépharose-4B indique une masse moléculaire apparente comprise entre 800.000 et 1.000.000 (Figure 1). Les chromatographie en phase gazeuse des monosaccharides libérés par hydrolyse et par méthanolyse montrent que le polysaccharide est constitué de galactose, glucose de N-acétylgalactosamine dans les rapports molaires 2:1:1 respectivement. Les rapports molaires en éthers méthyliques obtenus aprés méthanolyse et acétylation du polysaccharide perméthylé sont donnés dans le tableau 1. Ce résultat indique que le polysaccharide est constitué d'unité de répétition tétrasaccharidique est laquelle un résidu de galactose externe est branché sur la molécule de glucose en liaison 1-6.

Les spectres de résonance magnétique nucléaire du C-13 confirme la présence d'unités tétrasaccharidiques de répétition dans le polysaccharide par l'identification de 24 carbones (figure 2). Les carbones anomériques à 106.28 ppm et 105.92 ppm correspondent aux carbones 1 en configuration β d'un résidu de galactose et du résidu de glucose respectivement.

Le carbone anomérique à 101.02 ppm correspond au C-1 en configuration α du résidu de galactose et le carbone anomérique à 97.23 ppm correspond au C-1 en configuration α de la N-acétylgalactosamine. D'autre part, l'identification des signaux à 176.88 ppm et à 24.92 ppm, correspondant au groupement acétamido, confirme la présence d'une osamine dans l'unité de répétition du polysaccharide. Sur la base des résultats de la composition en monosaccharides, de la perméthylation et de la résonance magnétique nucléaire du C-13, nous pouvons déduire la structure suivante pour l'unité tétrasaccharidique de répétition du polysaccharide:

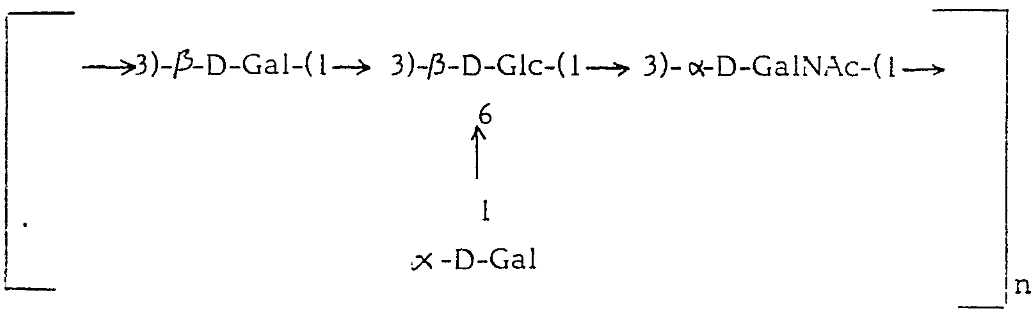

Cette formule signifie que dans l'unité de répétition tétrasaccharidique, le glucose est en configuration anomérique β sur le carbone en position 1. Le galactose lié sur le carbone en position 3 du glucose est en configuration anomérique β sur son carbone en position 1. Le galactose lié au carbone en position 6 du glucose présente une configuration anomérique α sur son carbone en position 1. Le N-acétylgalactosamine lié sur le carbone en position 1 du glucose présente une anomérie α sur son carbone en position 1. Le galactose à configuration β est lié au glucose par son carbone en position 1. Le galactose en configuration anomérique α sur son carbone en position 1 est lié au glucose par ce même carbone. Le N-acétylgalactosamine est lié au glucose par son carbone en position 3.

TABLEAU 1

RAPPORTS MOLAIRES EN ÉTHERS MÉTHYLIQUES OBTENUS PAR MÉTHANOLYSE DU POLYSACCHARIDE PERMÉTHYLÉ.

| 2.3,4,6-Me-$_4$-Gal | 2,4,6-Me$_3$-Gal | 2,4-Me$_2$-Glc | 4,6-Me$_2$-Gal-NAcNMe |
|---|---|---|---|
| 1.3 | 1.34 | 1 | 0.88 |

9. Essai d'identification et de dosage du polysaccharide par chromatographie liquide haute performance (HPLC)

Le protocole expérimental d'obtention du polysaccharide tel que décrit ci-dessus permet de préparer des quantités intéressantes d'hydrocolloides, mais il est trop long pour être appliqué à des études comparatives de production de polysaccharides par des souches différentes. C'est pourquoi a été mise au point une nouvelle méthode d'identification et de dosage du polysaccharide dans le yaourt. Des résultats intéressants ont été obtenus par chromatographie liquide haute performance, de yaourt sur colonne couplée d'échangeurs d'anions et de gel filtration.

A. Matériels et Méthodes.

1) Préparation de l'échantillon

10 ml de lait reconstitué stérilisé ensemencé par une souche de streptocoque SODIMA 822 (I-735) sont soniqués pendant 15 minutes à l'aide d'un appareil de sonication "Sonifer cell disruptor B-30" équipé d'une microsonde (pulsation intermittente à 50 % toutes les secondes).

Le produit obtenu est filtré successivement à l'aide de seringue Hamilton sur filtres 0,45 µm (2 fois, Millex-HA 0,45 µm), 0,22 µm (Millex-GS 0,22 µm) et Sep-Pak (C-18 cartridges for rapid preparation, Waters).

2) Chromatographie liquide haute pression

400 µl de l'échantillon sont injectés sur un échangeur d'anion (AX-300 Aquapore anion, 250x4,6 mm, Brownlee Labs) couplé à une colonne de gel filtration (Bio-Sil TSK-250, 300x7,5 mm, Bio-Rad). Les colonnes sont équilibrées dans un tampon phosphate 0,05 M, pH 6,5. L'élution est réalisée par ce tampon à un débit de 1 ml/min (HPLC Spectra Physics 8700, pression 60 bars). Toutes les trois injections, les protéines retenues par l'échangeur d'anions sont décrochées par le même tampon phosphate additionné de 0,5 M en chlorure de sodium, La détection est réalisé en UV à 206 nm (LDC/Milton Roy, atténuation 0,05) et par réfractométrie (Spectra Physics 6040, atténuation 4).

B. Résultats.

La figure 3 illustre les résultats HPLC de l'échantillon de lait reconstitué, soniqué et filtré. Le pic de temps de rétention 7,1 min correspond au polysaccharide, qui n'est pas retenu par l'échangeur d'anions et qui est exclu par la colonne de gel filtration. Ce pic possède le même temps de rétention que celui obtenu par chromatographie HPLC du polysaccharide isolé après hydrolyse pronasique et gel filtration.

Afin de vérifier l'identité de composition entre le polysaccharide isolé par la méthode classique et celui identifié par HPLC, nous avons réalisé des chromatographie semi-préparative en HPLC selon le protocole expérimental décrit ci-dessus.

Les dosages en monosaccharides des polysaccharides ainsi obtenus sont donnés dans le tableau suivant :

| polysaccharides | | monosaccharides | | |
|---|---|---|---|---|
| | | Gal | Glc* | GalNAc |
| Polysaccharide obtenu à parrir de la souche SODIMA 53 (I-733) | Préparation classique | 2.16 | 1 | 0.92 |
| | Préparation HPLC | 2.19 | 1 | 0.92 |
| polysaccharide obtenu par HPLC à partir streptocoque souche SODIMA 822 (I-735) | | 1.91 | 1 | 0.77 |

* calculés sur la base d'un résidu de glucose.

Les conclusions qui peuvent être tirées de ces résultats sont les suivantes :

- L'identification du polysaccharide produit par la bactérie, par HPLC du yaourt après sonication et filtration est possible.
- La macromolécule préparée de cette façon possède une composition en sucre identique à celle obtenue par le procédé ancien (2:1:1).
- Une application de la méthode à l'identification du polysaccharide excrété par une autre souche de Streptocoque permet de conclure à l'identité de composition en monosaccharides des deux polysaccharides obtenues par deux souches de Streptococcus Thermophilus.

EXEMPLE 2 : Propriétés épaississantes du polysaccharide.

I- Objectif : Déterminer la viscosité intrinsèque $[\eta]_0$ de la molécule polysaccharide, c'est-à- dire le volume hydrodynamique qu'occupe la macromolécule dans un solvant donné.

II - Matériels et Méthodes

La détermination de $[\eta]_0$ nécessite des mesures de la viscosité apparente $\eta_0$ à plusieurs niveaux de concentration.

La viscosité intrinsèque est alors déterminée selon l'équation (1)

$$\frac{\eta_{Sp_0}}{C} = [\eta]_0 + \lambda [\eta]_0^2 \cdot C \qquad (1)$$

avec

$$\eta_{Sp_0} = \frac{\eta_0 - \eta_{sol}}{\eta_{sol}}$$

avec $\eta_0$ = viscosité apparente de la solution
$\eta_{sol}$ = viscosité apparente du solvant
C = concentration de la macromolécule dans le solvant et
$\eta_{Sp_0}$ = viscosité spécifique
$\lambda$ = coefficient de HUGGINS.

La détermination de $\eta_0$ a été réalisée grâce à un rhéomètre à basse vitesse de cisaillement ($\dot{\gamma}$) le "Low shear 30" (CONTRAVES).

Le solvant utilisé est un tampon citrate-phosphate de pH 4.4.

Les concentrations respectivement utilisées de la macromolécule sont 5000 ppm (0,5 %) et 3333 ppm

(0.33 %) et 1000 ppm (0,1 %).

III - Résultats

Les rhéogrammes $\eta_0$ en fonction de $\dot{\gamma}$ permettent de déterminer les zones newtoniennes d'écoulement.

Sur le tableau I ci-après, sont réunis pour les trois différentes concentrations les valeurs de la viscosité spécifique en fonction de la concentration de la solution de polysaccharides.

TABLEAU I

| Concentration | C (%) | 0,100 | 0,333 | 0,500 |
|---|---|---|---|---|
| Viscosité spécifique | $\eta Sp_o/c$ (%)-1 | 2,3 | 3,87 | 4,7 |

Dans ces conditions, la viscosité intrinsèque du polysaccharide est de 1,75 dl/g.

IV - Conclusions

Le nouveau polysaccharide, isolé dans les conditions décrites, possède une viscosité intrinsèque de 1,75 dl/g qui est donc supérieure par exemple à celle de l'amylose épaississant exogène pour desserts lactés qui est de 1,54 dl/g dans KCl 0,3 M d'après MITCHELL, 1979.

EXEMPLE 3 - Propriété d'un yaourt contenant le polysaccharide.

I - Objectif : Tester le pouvoir épaississant du polysaccharide isolé tel que décrit, séché et remis en solution dans un yaourt à boire.

II - Matériel et Méthodes

Le polysaccharide isolé est mis en solution dans un tampon citrate-phosphate à pH4. On prépare une solution mère à 0,5 %.

On ajoute cette solution mère dans le yaourt liquide de façon à atteindre une concentration finale de polysaccharide, ajouté de 0,025 %.

A titre de témoin, on ajoute une même quantité de tampon dans du yaourt à boire.

La composition du yaourt à boire était la suivante :

| | | |
|---|---|---|
| lait entier | : | 97 % |
| ferment lactique | : | 3 % . |

Les mesures de viscosité sont réalisées au rhéomètre "Low Shear 30" (CONTRAVES).

III - Résultats

La viscosité du yaourt contenant le polysaccharide était plus élevée par rapport à celle du yaourt ne contenant pas le polysaccharide. Ainsi, on observe, pour une vitesse de cisaillement de 6 $s^{-1}$, que la viscosité du yaourt additionné de polysaccharide est égale à 285 mPa.s, alors que celle du témoin n'était que de 215 mPa.s (cf. figure 4).

Alors même que la quantité de polysaccharide ajouté était très faible (0,025 % en poids) ce qui donne une augmentation de viscosité très suffisante pour des quantités envisagées dans les yaourts de 0 à 0,5 %.

EXEMPLE 4 : MISE EN OEUVRE D'UN REACTEUR ENZYMATIQUE ULTRAFILTRATION POUR L'ISOLEMENT DE L'EXOPOLYSACCHARIDE DU PRODUIT PAR STREPTOCOCCUS THERMOPHILUS SUR LAIT RECONSTITUE STERILISE SELON L'INVENTION

Afin d'isoler les quantités significatives de polysaccharide, on a modifié le protocole expérimental d'isolement du polysaccharide à partir du milieu de culture. Ce nouveau procédé utilise l'hydrolyse protéolytique en continu dans un réacteur enzymatique approvisionné en substrat et couplé à une unité d'ultrafiltration qui élimine les produits d'hydrolyse et concentre le polysaccharide. L'action de l'enzyme est maintenue par l'apport de lait fermenté en solution dans le tampon d'hydrolyse.

A) Protocole expérimental

1) hydrolyse protéolytique et ultrafiltration des produits d'hydrolyse 30 gr de poudre de lait sont dissous dans 3 litres de tampon acétate de calcium 10 mM à pH 8. L'hydrolyse est effectuée par la pronase (Calbiochem, 500.000 units, Enz/substrat : 1/400) à 37° C en présence de toluène pour inhiber la croissance bactérienne.

Après 3 heures de réaction, l'hydrolysat est ultrafiltré à l'aide d'un appareil d'ultrafiltration Minitan

(Millipore), jeu de 10 plaques PTGC, seuil de coupure à 10.000.

Afin de maintenir le niveau constant, le réacteur est alimenté grâce à une pompe (Gilson) par une solution de poudre de yaourt à la concentration de 10 % dans le même tampon d'hydrolyse.

2) Montage

Le schéma du réacteur enzymatique couplée à la cellule d'ultrafiltration est donné dans la figure 5. Sur cette figure

1 = réacteur enzymatique : yaourt dans tampon acétate de calcium 10 mM pH 8 à 37° C, pronase .
2 = pompe d'alimentation des membranes d'ultrafiltration.
3 = membranes d'ultrafiltration, 10 plaques PTGC.
4 = pHmétre commandant la burette d'alimentation en NaOH.
5 = burette de NaOH 1.5 N.
6 = enregistreur de la chute de burette.
7 = pompe d'alimentation en substrat du réacteur enzymatique.
8 = réservoir : solution de poudre de yaourt à 10 p.cent dans le tampon d'hydrolyse

3) Purification du polysaccharide

Le rétentat est traité par de l'acide trichloroacétique à 10 % v/v qui précipite l'enzyme. Après précipitation, le surnageant est neutralisé par de l'ammoniaque et le polysaccharide précipité par 3 volumes d'éthanol. Le précipité éthanolique est repris par un tampon trisHCl 50 mM pH 8.6 est chromatographié sur colonne de DEAE tris-acryl (0.33 x 39 cm). Le polysaccharide non retenu est élué de la colonne par le même tampon. Les molécules retenues sont décrochées par le même tampon d'élution additionné de chlorure de sodium 1M. Après dialyse et lyophilisation, le pic polysaccharidique est chromatographié sur colonne de Sépharose 4B (92 x 1.8 cm) dans un tampon phosphate 5 mM mH 6.15. Le polysacharide est de nouveau dialysé et lyophilisé.

B) Résultats

L'hydrolyse est suivie par la chute de burette, la courbe est donnée à la figure 6.

Au bout de 3 heures, la vitesse d'hydrolyse décroît (plateau) ce qui traduit une diminution de la concentration en substrat.

L'ultrafiltration des produits d'hydrolyse ainsi que l'approvisionnement du réacteur en substrat (débit de 14 ml/min) permet de maintenir constante la vitesse d'hydrolyse de celui-ci. Ce phénomène est traduit par l'allure de la courbe obtenue. Le polysaccharide de masse moléculaire 1.000.000 (supérieur au seuil de coupure des plaques d'ultrafiltration) se concentre dans le rétentat qui s'enrichit en cette macromolécule. On a pu ainsi traiter 450 gr de poudre de yaourt en 50 heures. Le rétentat obtenu est ensuite diafiltré en maintenant le volume constant par addition de 4 volumes d'eau distillée puis concentré à 500 ml par ultrafiltration.

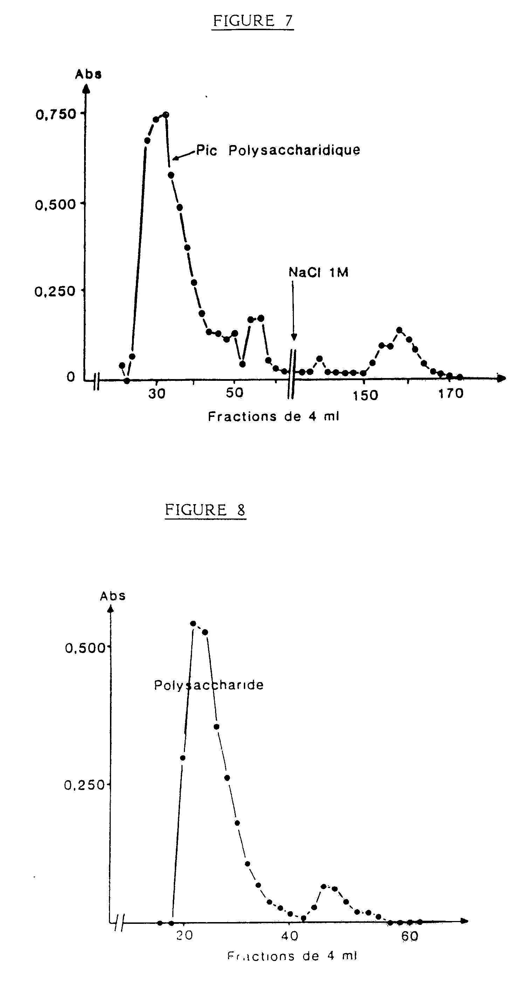

Après l'élimination de l'enzyme par précipitation trichloroacétique, le matériel macromoléculaire est isolé par précipitation à l'éthanol (3 volumes). Nous avons isoler ainsi 6.02 gr de précipité éthanolique à partir de 450 gr de poudre de yaourt. La purification du polysaccharide par chromatographie échangeuse d'ions sur DEAE tris-acryl (6 x 1 gr) (figure 7) suivie d'une chromatographie de gel filtration sur Sépharose 4B (figure 8) a permis de préparer 338 mg de polysaccharide.

Par rapport au premier protocole expérimental cette nouvelle méthodologie permet de raccourcir le temps de préparation de trois semaines pour le traitement de trois fois plus de substrat de départ (450 gr pour le nouveau protocole, 150 gr dans l'ancien protocole).

D'autre part, les rendements en polysaccharide purifié calculés sur la base des quantités de poudres de lait traités sont doublés : 0.075 % pour le nouveau protocole, 0.036 % pour le protocole initial.

EXEMPLE 5 : PROPRIETES ANTITUMORALES DU POLYSACCHARIDE ESTIMEES PAR L'ETUDE DE LA REGRESSION DE CELLULES TUMORALES D'ASCITE (Sarcome-180) CHEZ LA SOURIS APRES 30 JOURS DE TRAITEMENT

Activité antitumorale

Le polysaccharide a été inoculé chaque jour à des souris après inoculation de la tumeur de J=1 à J + 11 à des doses de 0,2 à 5 mg dans du liquide physiologique, injecté par voie intrapéritonéale.

A J+30, pour des doses de 0,2 à 5 mg/kg de polysaccharide la tumeur est inhibée de 32 à 73 % respectivement. Ce pourcentage d'inhibition est obtenue à l'aide du calcul suivant : (C-T/C) x 100

C : poids des tumeurs du groupe témoins.

T : poids des tumeurs du groupe traités par le polysaccharide.

| Substance | dose mg/kg par jour | poids moyen des tumeurs (g) | Inhibition[a] % | Régression complète[b] |
|---|---|---|---|---|
| Contrôle | - | 8.80 | - | 0/14 |
| Polysaccharide | 0.2 | 5.95 | 32 | 2/10 |
| | 1 | 4.61 | 48 | 0/7 |
| | 5 | 2.37 | 73 | 4/10 |

a = C-T/C) x 100 ; C = poids moyen de la tumeur des souris de contrôle

T = poids moyen de la tumeur des souris traitées

b = Nombre de souris dépourvues de tumeur/nombre de souris de contrôle ou traitées selon les cas.

REFERENCES

1 - J.P. Zanetta, W.C. Brekenridge et G. Vincendon, 1972, J. Chromatogr., 69,291-304.

2 - J.Paz-Parente, P.Cardon, Y.Leroy, J.Montreuil, B.Fournet et G.Ricart, 1984, Carbohydr.Res, 141,245-258.

3 - B.Fournet, G.Strecker, Y.Leroy et J.Montreuil, 1981, Anal.Biochem, 116,845-858.

**Revendications**

1. Polysaccharide caractérisé en ce qu'il est constitué par la répétition d'une unité tétrasaccharidique composée de galactose, glucose de N-acétygalactosamine dans les rapports molaires 2:1:1 respectivement et de masse moléculaire comprise entre 800.000 et 1 million.

2. .Polysaccharide selon la revendication 1, caractérisé en ce qu'il répond à la formule générale :

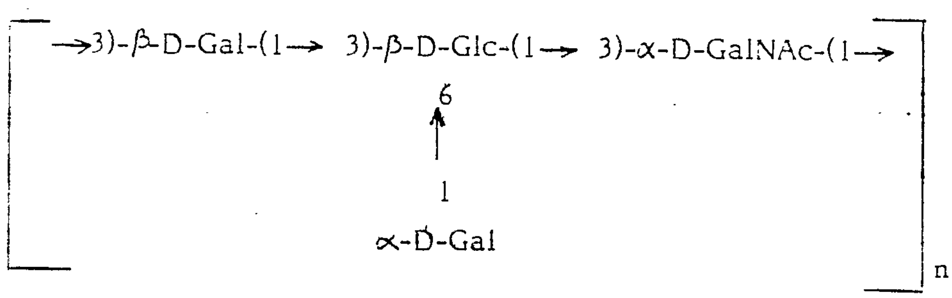

3. Procédé de production du polysaccharide selon l'une des revendications 1 ou 2, caractérisé en ce qu'il est produit dans un milieu de culture constitué de lait contenant de 1 à 5 %, de préférence 3 % en poids de ferment lactique de l'espèce Streptococcus thermophilus, à une température comprise entre 30 et 45° C, de préférence 40 à 45° C pour une durée de 3 à 5 heures puis de polysaccharide est isolé du milieu de culture.

4. Procédé selon la revendication 3, caractérisé en ce que le lait est du lait naturel ou du lait reconstitué.

5. Procédé selon l'une des revendications 3 ou 4, caractérisé en ce que la souche bactérienne utilisée a été sélectionnée en fonction de son caractère épaississant.

6. Procédé selon l'une des revendications 3 à 5, caractérisé en ce que le polysaccharide est produit à partir de l'une des souches bactériennes Streptococcus thermophilus référencée CNCM I-733, CNCM I-734 et CNCM I-735 mise en culture dans du lait reconstitué stérilisé avec un inoculum de 3 % en poids de ferment Streptococcus thermophilus, la fermentation étant réalisée à 43° C jusqu'à pH 5, soit environ 4 heures.

7. Procédé selon l'une des revendications 3 à 6, caractérisé en ce que le polysaccharide est isolé du milieu de culture par une hydrolyse protéolitique en continu dans un réacteur enzymatique, l'action de l'enzyme étant maintenu par l'apport de lait fermenté en solution dans le tampon d'hydrolyse, puis par élimination des produits d'hydrolyse et concentration du polysaccharide à l'aide d'une unité d'ultrafiltration.

8. Utilisation du polysaccharide selon l'une des revendications précédentes, à titre d'agent de texture épaississant.

9. Utilisation du polysaccharide selon l'une des revendications précédentes comme médicament à titre

d'agent antitumoral.

10. Procédé d'épaississement d'un produit du type lait fermenté, notamment de yaourt, caractérisé en ce qu'on ajoute au dit produit un polysaccharide selon l'une des revendications précédentes.

11. Procédé selon la revendication 10, caractérisé en ce que la quantité de polysaccharide ajoutée est comprise entre 0,1 et 0,5 % en poids du produit.

FIG_1

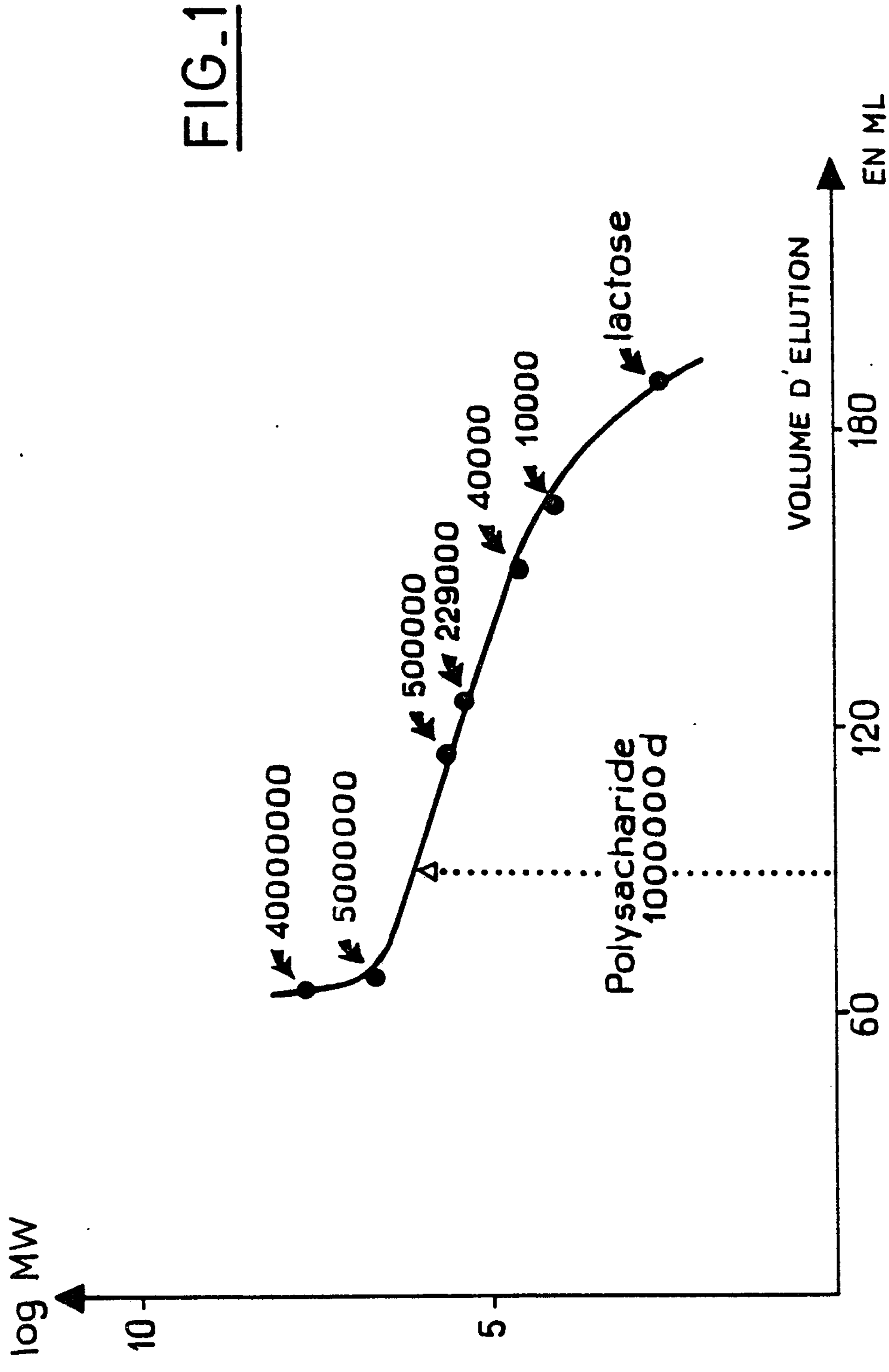
log MW
10
5
60
120
180
VOLUME D'ELUTION
EN ML
40000000
50000000
500000
229000
40000
10000
lactose
Polysacharide
1000000 d

FIG. 2

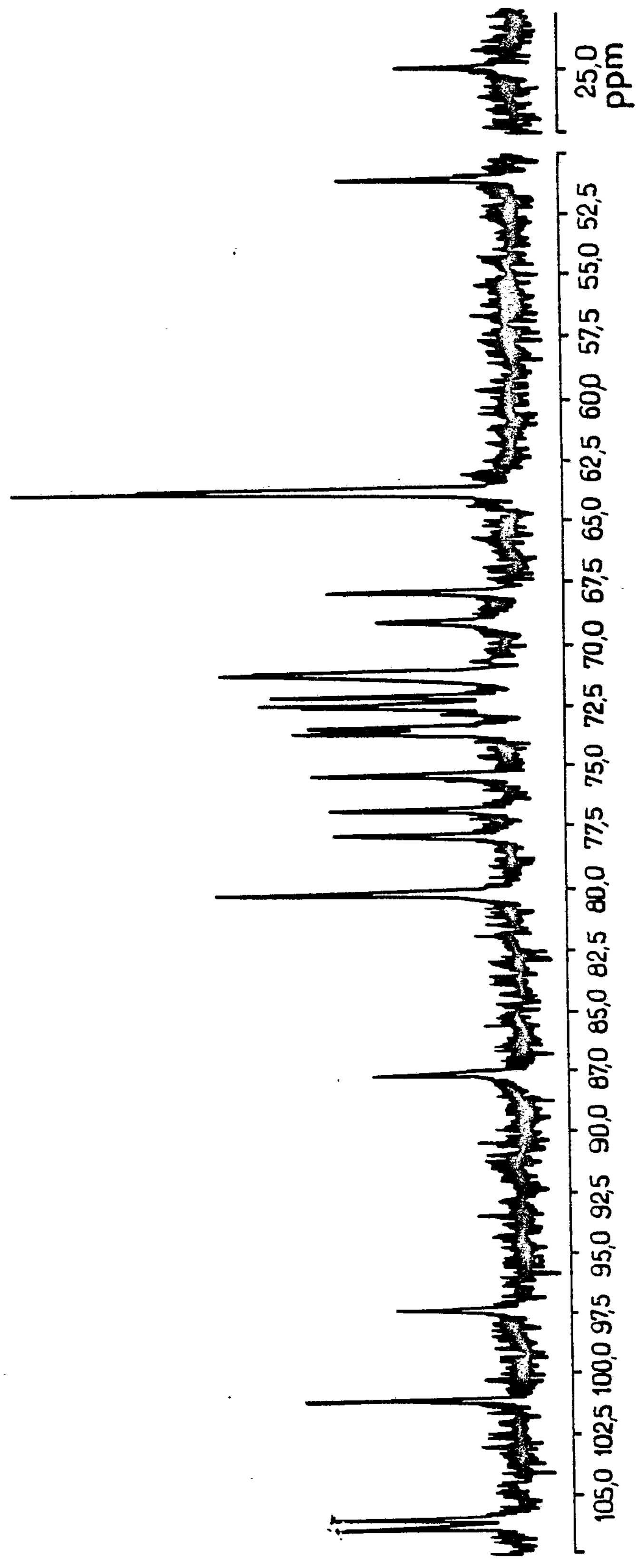
25,0
ppm
52,5
55,0
57,5
60,0
62,5
65,0
67,5
70,0
72,5
75,0
77,5
80,0
82,5
85,0
87,0
90,0
92,5
95,0
97,5
100,0
102,5
105,0

FIG.3

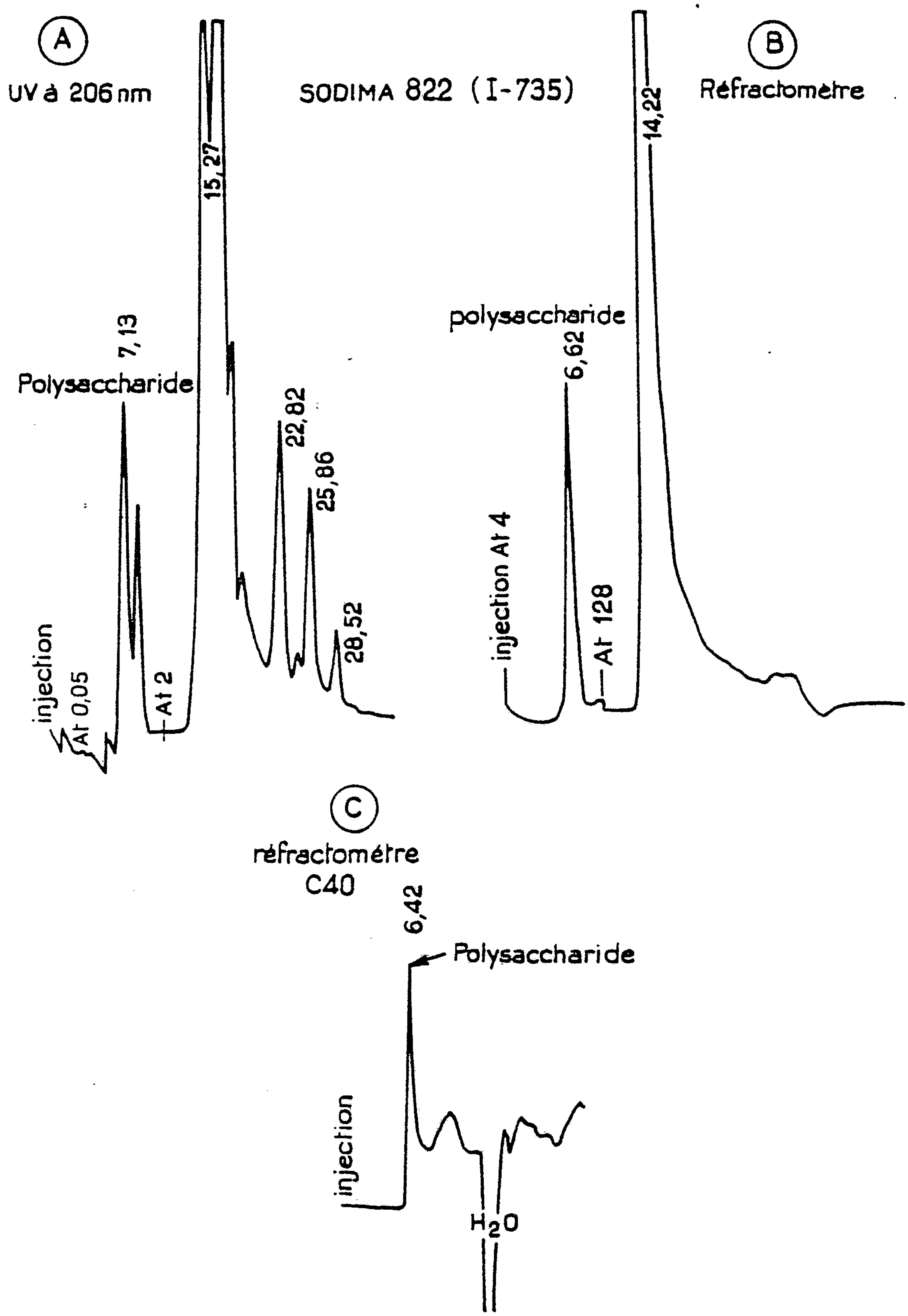
A
UV à 206nm
SODIMA 822 (I-735)
B
Réfractomètre
15,27
7,13
Polysaccharide
22,82
25,86
28,52
injection
At 0,05
At 2
14,22
polysaccharide
6,62
injection At 4
At 128
C
réfractomètre
C40
6,42
Polysaccharide
injection
$H_2O$

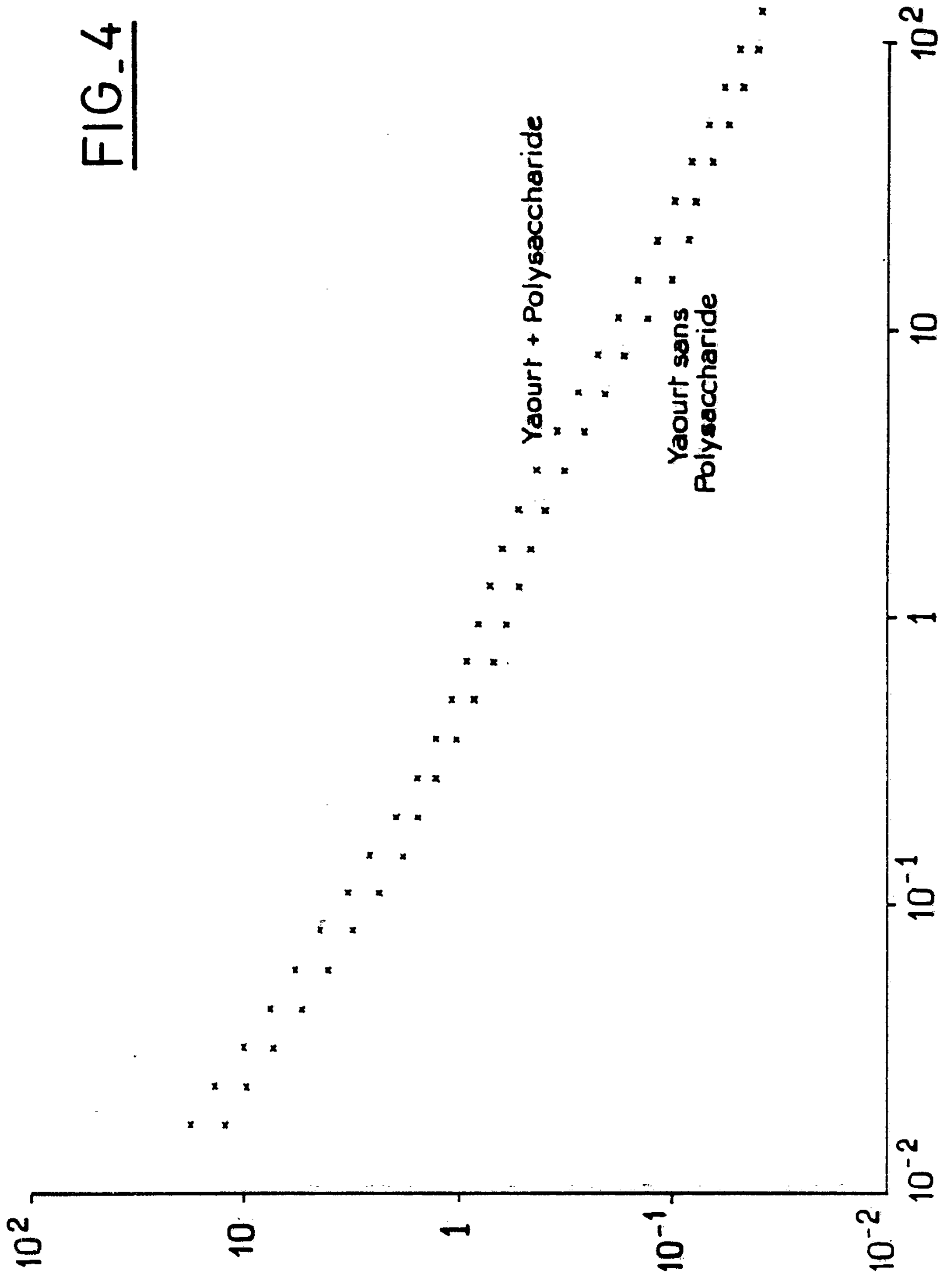
Yaourt + Polysaccharide
Yaourt sans
Polysaccharide
10²
10
1
10⁻¹
10⁻²
10⁻²
10⁻¹
1
10
10²

FIG_4

FIGURE 5

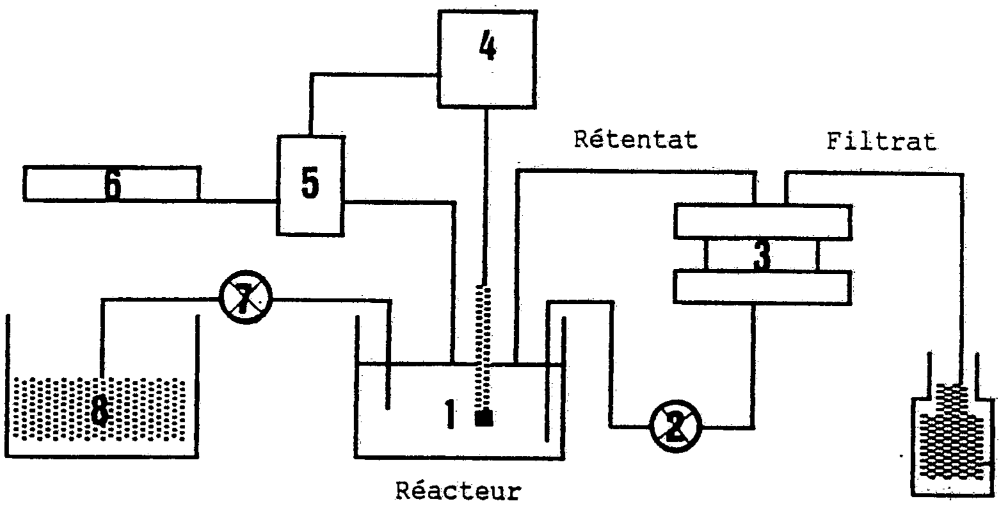


FIGURE 6

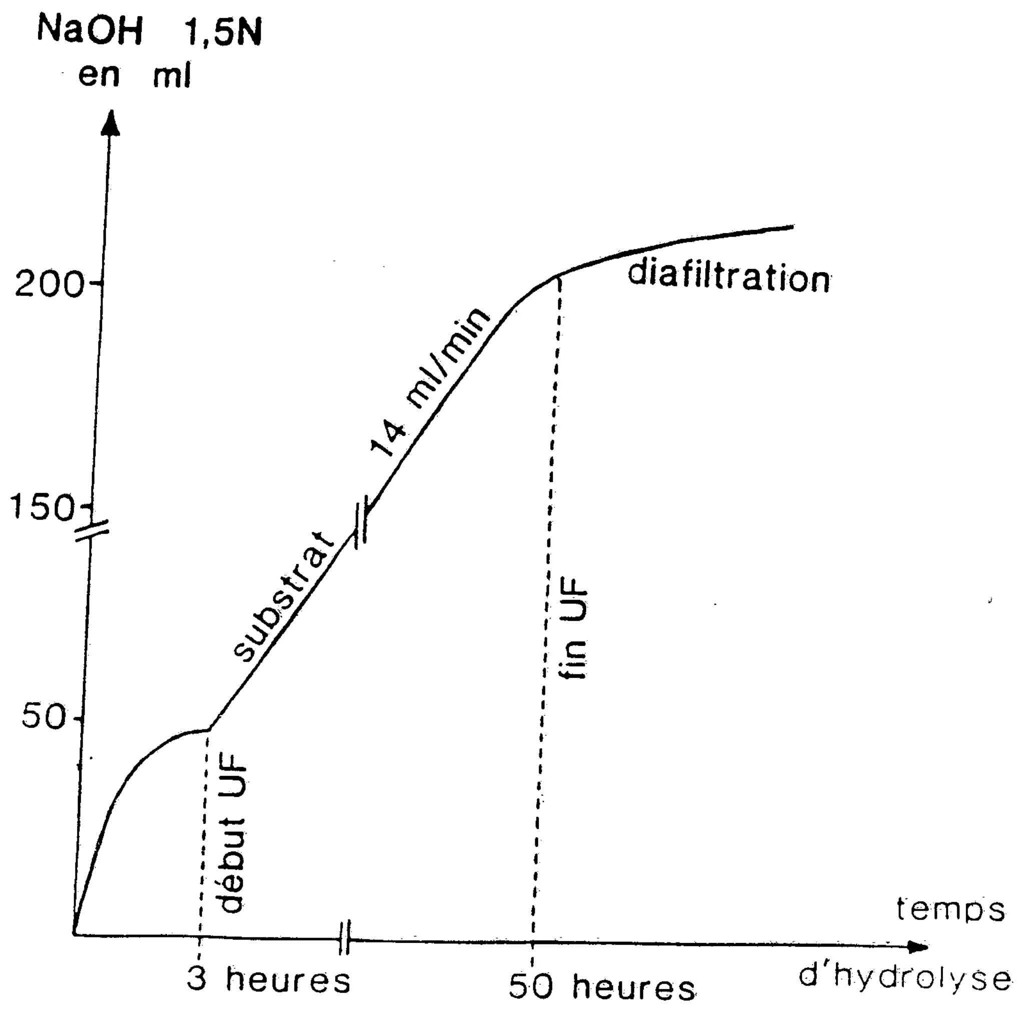

FIGURE 7

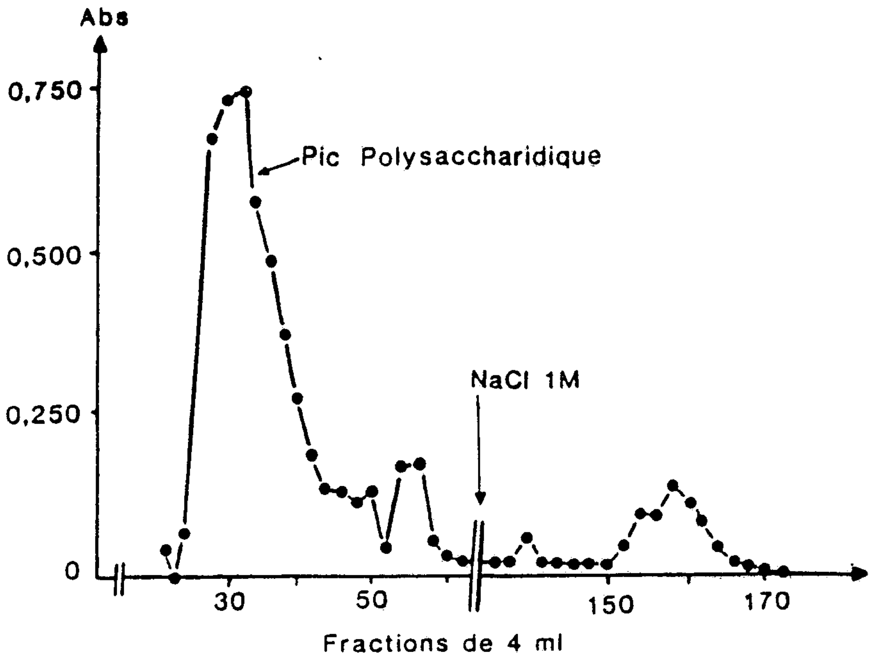


FIGURE 8